Europäisches Patentamt

European Patent Office

Office européen des brevets

(19)

(11) EP 0 600 309 B1

(12) EUROPÄISCHE PATENTSCHRIFT

(45) Veröffentlichungstag und Bekanntmachung des Hinweises auf die Patenterteilung:
03.09.1997 Patentblatt 1997/36

(51) Int. Cl.$^6$: C07C 203/00, C07C 201/04

(21) Anmeldenummer: 93118554.0

(22) Anmeldetag: 18.11.1993

(54) **Verfahren zur kontinuierlichen Herstellung von Alkylnitriten**

Process for the continuous preparation of alkylnitrites

Procédé de préparation continu de nitrites d'alkyle

(84) Benannte Vertragsstaaten:
BE DE ES FR GB IT NL

(30) Priorität: 01.12.1992 DE 4240311

(43) Veröffentlichungstag der Anmeldung:
08.06.1994 Patentblatt 1994/23

(73) Patentinhaber: BAYER AG
51368 Leverkusen (DE)

(72) Erfinder:
• Landscheidt, Heinz, Dr.
D-47067 Duisburg (DE)
• Wagner, Paul, Dr.
D-40597 Düsseldorf (DE)
• Klausener, Alexander, Dr.
D-52223 Stolberg (DE)

(56) Entgegenhaltungen:
US-A- 2 739 166

**Beschreibung**

Die vorliegende Erfindung betrifft ein Verfahren zur Herstellung von Alkylnitriten aus Stickstoffdioxid und Alkoholen in einer im Gegenstrom betriebenen Kolonne.

Alkylnitrite (Alkylester der salpetrigen Säure) finden vielfältige Verwendung, so beispielsweise als Zusatzstoffe für Motoröle, als Stabilisatoren für ungesättigte organische Verbindungen, als Spasmolytika, als Reagenzien für Oximierungen, Nitrosierungen und Diazotierungen sowie als Hilfsstoffe für chemische Synthesen.

Der gebräuchlichste, im Laboratorium leicht durchführbare Weg zur Herstellung von Alkylnitriten stellt die Umsetzung von Natriumnitrit mit starken Säuren, beispielsweise mit Schwefelsäure, in Gegenwart des betreffenden Alkohols dar:

$$2\ NaNO_2 + 2\ ROH + H_2SO_4 \rightarrow 2\ RONO + Na_2SO_4 + 2H_2O \tag{1}$$

Das Verfahren nach Gleichung (1) beruht auf der hohen Veresterungsgeschwindigkeit der salpetrigen Säure mit dem Alkohol, wobei die Reaktionsbedingungen so gewählt werden können, daß das entstehende Alkylnitrit dem Reaktionsgleichgewicht gasförmig, gegebenenfalls durch Abdestillieren, entzogen werden kann. Die Nachteile dieses Verfahrens liegen jedoch in der Verwendung teurer Ausgangsmaterialien, wie beispielsweise Natriumnitrit, sowie im Zwangsanfall großer Mengen nicht verwendbarer anorganischer Salze, insbesondere von Natriumsulfat. Darüberhinaus gelingt es im allgemeinen nicht, den eingesetzten Alkohol vollständig zur Reaktion zu bringen, so daß entweder entsprechende Verluste in Kauf genommen werden müssen oder eine aufwendige Aufarbeitung des anfallenden Abwassers durchgeführt werden muß.

Ein anderes Verfahren zur Herstellung von Alkylnitriten basiert auf der Verwendung von Stickoxiden. Dabei werden bevorzugt Stickstoffmonoxid, Sauerstoff und der betreffende Alkohol miteinander zur Reaktion gebracht, wobei die folgenden Reaktionen ablaufen:

$$2\ NO + O_2 \rightarrow 2\ NO_2 \tag{2}$$

$$NO + NO_2 \leftrightarrow N_2O_3 \tag{3}$$

$$ROH + N_2O_3 \rightarrow RONO + HNO_2 \tag{4}$$

$$ROH + HNO_2 \rightarrow RONO + H_2O \tag{5}$$

$$N_2O_3 + H_2O \rightarrow 2\ HNO_2 \tag{6}$$

Man versucht hierbei, die Herstellung der angestrebten Alkylnitrite möglichst nur nach den Reaktionsgleichungen (2) bis (5) ablaufen zu lassen, wobei als einziger Abfallstoff Wasser erhalten wird. Jedoch ist die in Reaktionsgleichung (6) wiedergegebene Reaktion im allgemeinen nicht vermeidbar, da das nach Reaktionsgleichung (3) gebildete Distickstofftrioxid nicht nur mit dem Alkohol im Sinne der Gleichung (4), sondern auch mit dem nach Gleichung (5) entstandenen Wasser abreagieren kann. In Gegenwart ausreichender, insbesondere überschüssiger Mengen des Alkohols wird jedoch die dabei entstehende salpetrige Säure im Sinne der Reaktionsgleichung (5) unter Bildung des gewünschten Alkylnitrits und Wasser abgefangen und geht somit nicht als Abfallstoff verloren. Ein nach den geschilderten Reaktionen ablaufendes Verfahren wird beispielsweise in US 2.831.882 beschrieben, wo zur Herstellung von Isopropylnitrit Stickstoff, Stickstoffmonoxid, Stickstoffdioxid und Isopropanol in ein Reaktionsgefäß eingeleitet werden. DE-PS 11 56 775 beschreibt die Herstellung von Methylnitrit durch Mischen von Stickstoffmonoxid und Sauerstoff im Verhältnis von 4,4:1 und Einleiten des so erhaltenen Gasgemisches in flüssiges Methanol. In US 4.353.843 wird die Umsetzung eines Gemisches aus Stickstoffmonoxid und Stickstoffdioxid, wobei der Anteil des Stickstoffmonoxids größer ist als der des Stickstoffdioxids, mit gasförmigem Methanol beschrieben. In EP 310 191 schließlich wird die Bildung niedriger Alkylnitrite durch Reaktion von Stickstoffmonoxid, Sauerstoff und den entsprechenden Alkoholen in einer speziellen, eine Reaktions- und eine Rektifikationszone enthaltenden Apparatur beschrieben, wobei das im Zuge der Alkylnitritherstellung anfallende Wasser kontinuierlich durch Auswaschen aus dem aufsteigenden Produktgasstrom entfernt wird. Nachteilig bei diesem Verfahren ist der Einsatz des in reiner Form technisch schlecht verfügbaren Stickstoffmonoxids. Ein weiterer Nachteil besteht darin, daß zur Zurückdrängung der im Verfahren von EP 310 191 unerwünschten Bildung von Salpetersäure, die durch den Ablauf von Nebenreaktionen gemäß Gleichungen (7) bis (9) entsteht, im allgemeinen Überschüsse an Stickstoffmonoxid eingesetzt werden müssen:

$$2\ NO_2 \leftrightarrow N_2O_4 \tag{7}$$

$$ROH + N_2O_4 \rightarrow RONO + HNO_3 \tag{8}$$

2

$$N_2O_4 + H_2O \rightarrow HNO_2 + HNO_3 \tag{9}$$

In US 2.739.166 wird ein Verfahren beschrieben, demzufolge Alkylnitrite beim Durchleiten von gasförmigem Stickstoffdioxid durch die betreffenden Alkohole erhalten werden. Dabei erhält man, ebenfalls wieder nach den Reaktionsgleichungen (7) bis (8), das gewünschte Alkylnitrit im Gemisch mit Salpetersäure und nicht abreagiertem überschüssigem Alkohol. Grundsätzlich bringt eine derartige Vorgehensweise einen Vorteil, da sie die Verwendung des in reiner Form technisch besser verfügbaren Stickstoffdioxids ermöglicht. Die Exothermie der Umsetzung von Stickstoffdioxid und Alkoholen zu Alkylnitriten und Salpetersäure ist außerdem kleiner als die von Stickstoffmonoxid, Sauerstoff und Alkoholen zu Alkylnitriten und Wasser. Mit dem Wegfall der Notwendigkeit, Sauerstoff bereitzustellen und in Abhängigkeit von der Stickstoffmonoxidmenge zuzudosieren, ergeben sich damit auch geringere Probleme bei der Abfuhr der Reaktionswärme.

Nachteilig bei dem in US 2.739.166 beschriebenen Verfahren ist jedoch, daß im Verlaufe fortschreitender Umsetzungen in der flüssigen Phase Gemische aus Alkoholen und Salpetersäure entstehen, wobei sich die Konzentration der letzteren andauernd erhöht. Dies ist nicht nur unter sicherheitstechnischen Aspekten bedenklich, es muß darüberhinaus auch mit vermehrter Nebenproduktbildung, insbesondere mit dem Entstehen von toxischen und hochexplosiven Alkylnitraten, gerechnet werden. Weiter ist es von Nachteil, daß die bei der Herstellung der Alkylnitrite anfallenden Salpetersäure-Rückstände mindestens noch unumgesetzten Alkohol, häufig auch noch gelöstes Alkylnitrit sowie in Nebenreaktionen gebildetes Alkylnitrat enthalten und nicht ohne aufwendige Aufarbeitung weiterverwendet werden können. Dies stellt, bezogen auf das eingesetzte Stickstoffdioxid, eine wirtschaftlich unbefriedigende Situation dar.

Es bestand daher weiterhin die Aufgabe, ausgehend von technisch gut verfügbarem Stickstoffdioxid, ein Verfahren zur Herstellung von Alkylnitriten zu entwickeln, das sich insbesondere für die Synthese der technisch interessanten flüchtigen Verbindungen Methylnitrit und Ethylnitrit eignet, nicht zu unerwünschten Nebenprodukten, wie beispielsweise Alkylnitraten, führt und die anfallende Salpetersäure, im günstigsten Falle unter Vermeidung zusätzlicher Reinigungsmaßnahmen, in einer für weitere Verwendungen ausreichenden Qualität liefert. Wesentlich für das erfolgreiche Betreiben eines derartigen Prozesses ist die weitgehende Berücksichtigung folgender Forderungen:

- Stickstoffdioxid und der zur Reaktion gebrachte Alkohol sollen möglichst vollständig unter Bildung des gewünschten Alkylnitrits und Salpetersäure abreagieren.

- Reaktionsführung und Reaktionstechnik sind so zu gestalten, daß die unerwünschte Bildung von Nebenprodukten wie Alkylnitraten unterbleibt und das gewünschte Alkylnitrit in möglichst reiner Form erhalten wird.

- Die im Zuge der Umsetzung entstehende Salpetersäure soll ohne eine zusätzliche aufwendige Operation, wie beispielsweise eine Extraktion, vollständig und in einer für ihre weitere Verwendung ausreichenden Qualität von dem gewünschten Alkylnitrit abgetrennt werden.

- Die im Verlaufe der innerhalb des Reaktors ablaufenden Reaktionen freiwerdende Reaktionswärme ist abzuführen.

- Im Hinblick auf die sicherheitstechnische Anforderungen an die technische Durchführung des anzustrebenden Verfahrens müssen lokale Überhitzungen sowie das Entstehen zündfähiger Gemische vermieden werden.

Es wurde ein Verfahren zur Herstellung von $C_1$-$C_6$-Alkylnitriten durch Umsetzung von Stickstoffdioxid mit $C_1$-$C_6$-Alkoholen gefunden, das dadurch gekennzeichnet ist, daß man in einer im Gegenstrom betriebenen Kolonne den Alkohol oder ein Alkohol/Wasser-Gemisch in den oberen Teil der Kolonne einspeist und das Stickstoffdioxid oder ein Stickstoffdioxid/Inertgas-Gemisch in den unteren Teil der Kolonne einspeist und das entstehende Alkylnitrit als Kopfprodukt und die mitentstehende Salpetersäure als Sumpfprodukt der Kolonne entnimmt.

Das erfindungsgemäße Verfahren besitzt beispielsweise folgende Vorzüge:

- Es verwendet als Ausgangsmaterial das im Vergleich zu Stickstoffmonoxid technisch besser verfügbare Stickstoffdioxid, wie es beispielsweise in industriellen Anlagen zur Produktion hochkonzentrierter Salpetersäure anfällt.

- Es erlaubt die Verwendung von wasserhaltigen Alkoholen, ohne daß sich dies negativ auf die Qualität der hergestellten Alkylnitrite auswirkt; mit Hilfe des Wassergehaltes des zugesetzten Alkohols läßt sich der Verdünnungsgrad der am Boden der Kolonne gewonnenen Salpetersäure in weiten Grenzen gezielt einstellen.

- Es liefert die im Zuge der Reaktion gleichfalls entstehende Salpetersäure in hoher Qualität, so daß sie ohne aufwendige Reinigungen für weitere Umsetzungen verwendet werden kann.

- Es arbeitet ohne einen Zwangsanfall an anorganischen Salzen und führt nahezu ausschließlich zu Wertstoffen, was unter wirtschaftlichen und ökologischen Gesichtspunkten von großem Vorteil ist. Das Nebenproduktspektrum ist nach Zahl und Menge außerordentlich gering.

- Es eignet sich insbesondere zur Herstellung der technisch interessanten niedrigsiedenden Alkylnitrite, wie vor allem Methyl- und Ethylnitrit, bevorzugt von Methylnitrit.

- Es erlaubt, insbesondere bei Mitverwendung von Wasser und/oder Inertgas, durch die konsequente Vermeidung von zündfähigen und explosionsfähigen Stoffgemischen, Stoffkonzentrationen und kritischen Temperaturen die Einhaltung aller Sicherheitsanforderungen.

Im Vergleich mit dem aus US 2.739.166 bekannten Stand der Technik war es vor allem überraschend, daß das erfindungsgemäße Verfahren die Reaktionsprodukte, also das jeweils angestrebte Alkylnitrit und die gleichfalls entstehende Salpetersäure, in einer derart hohen Reinheit liefert, daß im allgemeinen keine weiteren aufwendigen Reinigungsoperationen erforderlich werden. Insbesondere gelingt es überraschend leicht, die Bildung von Nebenprodukten, wie beispielsweise von Alkylnitraten, praktisch vollständig zu unterdrücken.

Alkohole für das erfindungsgemäße Verfahren sind geradkettige und verzweigte $C_1$-$C_6$-Alkanole, bevorzugt geradkettige $C_1$-$C_4$-Alkanole und besonders bevorzugt Methanol und Ethanol. Insbesondere genannt sei Methanol.

Das molare Verhältnis des zudosierten Alkohols zum eingespeisten Stickstoffdioxid beträgt 0,3 bis 1:1, bevorzugt 0,4 bis 0,8:1 und besonders bevorzugt 0,5 bis 0,7:1.

Das molare Verhältnis von zudosiertem Wasser zum eingespeisten Stickstoffdioxid beträgt 0 bis 2:1, bevorzugt 0,2 bis 1,6:1 und besonders bevorzugt 0,8 bis 1:1. Die Untergrenze Null im allgemeinen Bereich zeigt an, daß das erfindungsgemäße Verfahren ohne zudosiertes Wasser durchführbar ist. Die von Null verschiedene Untergrenze in den Vorzugsbereichen zeigt an, daß das erfindungsgemäße Verfahren bevorzugt in Gegenwart von zudosiertem Wasser durchgeführt wird.

Der Druck, unter dem das erfindungsgemäße Verfahren ausgeübt wird, orientiert sich u.a. an den Stoffdaten und Phasengleichgewichten der beteiligten Komponenten, weiterhin an Überlegungen zur Wirtschaftlichkeit im Sinne des Erzielens einer maximal möglichen Raum-Zeit-Ausbeute und an den Anforderungen der Sicherheitstechnik. Während beispielsweise im Falle der höhersiedenden Alkylnitrite entweder unter vermindertem Druck und/oder auf höherem Temperaturniveau gearbeitet werden muß, um das gewünschte Alkylnitrit am Kopf der Reaktionskolonne abnehmen zu können, ist beispielsweise im Falle des niedrigsiedenden Methylnitrits eine Druckfahrweise von Vorteil. Grundsätzlich läßt sich demnach das erfindungsgemäße Verfahren sowohl bei Normaldruck als auch unter vermindertem oder erhöhtem Druck durchführen. Als Druckbereich kommt der von 0,5 bis 10, bevorzugt 0,8 bis 8, besonders bevorzugt 1 bis 5 bar in Frage.

Für die Temperatur, bei der das erfindungsgemäße Verfahren zur Ausführung gelangt, gelten ähnliche Überlegungen. Sie wird so gewählt, daß die Stoffdaten und Phasengleichgewichte der beteiligten Komponenten Berücksichtigung finden, daß eine Wirtschaftlichkeit in Sinne des Erzielens einer maximal möglichen Raum-Zeit-Ausbeute erfüllt wird und die Anforderungen der Sicherheitstechnik befriedigt werden. Während beispielsweise im Falle der höhersiedenden Alkylnitrite gegebenenfalls auf höherem thermischen Niveau gearbeitet werden muß, um das gewünschte Alkylnitrit am Kopf der Reaktionskolonne abnehmen zu können, werden beispielsweise im Falle des niedrigsiedenden Methylnitrit deutlich niedrigere Temperaturen gewählt. Im Sinne dieser Überlegungen werden auch Druck und Temperatur sinnvoll miteinander verknüpft. Als Temperaturbereiche kommen 0 bis 90°C, bevorzugt 10 bis 80°C in Frage.

Das erfindungsgemäß eingesetzte Stickstoffdioxid kann als solches oder in Form eines Gemisches mit Inertgasen verwendet werden. Solche Inertgase sind beispielsweise Stickstoff, Kohlendioxid, Argon, Kohlenmonoxid, bevorzugt Stickstoff und Kohlendioxid. Ein solches Inertgas kann separat oder als Gemisch mit Stickstoffdioxid in den Reaktor eingespeist werden. Das Volumenverhältnis (NI/NI) des Inertgases zum Stickstoffdioxid beträgt 0 bis 5:1, bevorzugt 0,1 bis 3:1 und besonders bevorzugt 0,2 bis 2:1.

Die Untergrenze Null des genannten Volumenverhältnisses zeigt an, daß das erfindungsgemäße Verfahren ohne Mitverwendung inerter Gase durchgeführt werden kann. Die von Null verschiedene Untergrenze in den Vorzugsbereichen zeigt an, daß bevorzugt in Gegenwart eines solchen Inertgases gearbeitet wird. Die Mitverwendung größerer Inertgasmengen, z.B. Inertgas : Stickstoffdioxid >2, im erfindungsgemäßen Verfahren ist insbesondere bei der Herstellung höherer Alkylnitrite von Vorteil.

Zur Durchführung des erfindungsgemäßen Verfahrens wird eine Reaktionskolonne eingesetzt, die eine große Phasengrenzfläche (gasförmig/flüssig) und eine innige Vermischung der flüssigen und der gasförmigen Phase gewährleistet. Dies kann durch den Einbau von Böden, wie Glockenböden, Sieblochböden, Ventilböden, Schlitzböden usw., wie sie für eine thermische Trennung üblich sind, durch Füllkörper aller Art, wie sie bei thermischen Trennoperationen üblich sind, oder durch die Ausrüstung der Kolonne mit ungeordneten Füllkörpern aller Art oder mit geordneten Packungen aus Metall, Keramik, Kunststoff, Glas oder weiteren Materialien, die gegenüber den Reaktionspartnern des erfindungsgemäßen Verfahrens inert sind, erreicht werden. Solche Kolonnen mit Einbauten, Füllungen oder Packungen sowie die Füllungen und Packungen selbst sind handelsüblich und dem Fachmann bekannt. In bevorzugter Weise finden strukturierte Packungen Verwendung. Die Belastung der Reaktionsapparatur, bezogen auf die gesamte eingespeiste Gasmenge (Stickstoffdioxid und gegebenenfalls Inertgas) beträgt 10 bis 3000, bevorzugt 20 bis 2000 und besonders bevorzugt 40 bis 1000 Nl/l freien Volumens in der Reaktionskolonne pro Stunde. Diese Verhältnisse gewährleisten eine ausreichende Verweilzeit, um einen möglichst vollständigen Umsatz zu erreichen.

Wird bei der Durchführung des erfindungsgemäßen Verfahrens Wasser zudosiert, so kann dieses separat oder im Gemisch mit dem Alkohol eingesetzt werden. In bevorzugter Weise wird der Alkohol oberhalb der Zudosierung des Wassers in die Kolonne eingeführt.

Der Produktstrom mit dem gewünschten Alkylnitrit wird der Reaktionskolonne gasförmig am Kopf entnommen. Der darin enthaltene Alkohol kann durch Kühlung und/oder durch Komprimieren des Produktstromes mindestens teilweise daraus entfernt werden; der so zurückgewonnene Alkohol kann in die Reaktion zurückgeführt werden. Weiterhin kann auch das im Kopfstrom enthaltene Alkylnitrit, das gegebenenfalls im Gemisch mit Alkohol vorliegt, durch Wäsche mit einem geeigneten Waschmedium, durch starke Kühlung und/oder durch Komprimieren des gasförmigen Produktstroms zumindest teilweise aus diesem entfernt werden; das dabei zurückbleibende Inertgas, falls ein solches mitverwendet wurde, kann in die Reaktion zurückgeführt werden.

Das erfindungsgemäß hergestellte Alkylnitrit kann für viele weitere Umsetzungen, wie für Nitrosierungen, Diazotierungen usw. direkt verwandt werden. Für den Fall, daß bei diesen Verwendungen das Alkylnitrit nicht von dem mitverwendeten Inertgas getrennt worden war, fällt dieses Inertgas nach den genannten Umsetzungen an und kann daraus in das erfindungsgemäße Verfahren zurückgeführt werden.

Beispiele

Beispiel 1

Ein Kohlendioxid-Gasstrom von 10 l/h wurde mit einem Stickstoffdioxidgasstrom von 2,5 l/h (0,112 Mol/h) gemischt, auf 25°C temperiert und am unteren Ende eines mit Raschig-Ringen befüllten und auf 25°C temperierten Rohres eingeleitet (Abmessungen: 1,6 cm Durchmesser, 50 cm Höhe).

Im Gegenstrom dazu wurden am oberen Ende des Reaktors 3 g/h Methanol und 3 g/h Wasser eingespeist.

Von dem am Kopf der Kolonne erhaltenen Gasstrom wurden Proben genommen, die hinsichtlich ihrer Zusammensetzung analysiert wurden.

| Ergebnis: | Kohlendioxid | 85 % |
|---|---|---|
| | Methylnitrit | 11 % |
| | Methanol | 4 % |

Das am unteren Auslauf der Kolonne anfallende flüssige Produkt wurde gesammelt und hinsichtlich seiner Zusammensetzung analysiert.

| Ergebnis: | Wasser | 45 % |
|---|---|---|
| | Salpetersäure | 55 % |

Methanol konnte nicht nachgewiesen werden.

Beispiel 2

Der in Beispiel 1 beschriebene Versuch wurde wiederholt, wobei der Gasstrom und das Reaktionsrohr statt auf 25°C auf 40°C temperiert wurde. Der gasförmige Kopfstrom wurde analysiert.

| Ergebnis: | Kohlendioxid | 84 % |
|-----------|--------------|------|
|           | Methylnitrit | 10 % |
|           | Methanol     | 6 %  |

Das am unteren Auslauf der Kolonne anfallende flüssige Produkt wurde gesammelt und hinsichtlich seiner Zusammensetzung analysiert.

| Ergebnis: | Wasser       | 45 % |
|-----------|--------------|------|
|           | Salpetersäure | 55 % |

Methanol konnte nicht nachgewiesen werden.

Beispiel 3

Ein Kohlendioxid-Gasstrom von 10 l/h wurde mit einem Stickstoffdioxidgasstrom von 7,5 l/h (0,335 Mol/h) gemischt, auf 25°C temperiert und am unteren Ende eines mit Raschig-Ringen befüllten und auf 25°C temperierten Rohres eingeleitet (Abmessungen: 1,6 cm Durchmesser, 50 cm Höhe).
Im Gegenstrom dazu wurden am oberen Ende des Reaktors 7 g/h Methanol und 7 g/h Wasser eingespeist.
Von dem am Kopf der Kolonne erhaltenen Gasstrom wurden Proben genommen, die hinsichtlich ihrer Zusammensetzung analysiert wurden.

| Ergebnis: | Kohlendioxid | 67 % |
|-----------|--------------|------|
|           | Methylnitrit | 27 % |
|           | Methanol     | 6 %  |

Das am unteren Auslauf der Kolonne anfallende flüssige Produkt wurde gesammelt und hinsichtlich seiner Zusammensetzung analysiert.

| Ergebnis: | Wasser       | 39 % |
|-----------|--------------|------|
|           | Salpetersäure | 61 % |

Methanol konnte nicht nachgewiesen werden.

Beispiel 4

Ein Kohlendioxid-Gasstrom von 20 l/h wurde mit einem Stickstoffdioxidgasstrom von 12,3 l/h (0,549 Mol/h) gemischt, auf 25°C temperiert und am unteren Ende eines mit Raschig-Ringen befüllten und auf 25°C temperierten Rohres eingeleitet (Abmessungen: 1,6 cm Durchmesser, 50 cm Höhe).
Im Gegenstrom dazu wurden am oberen Ende des Reaktors 12 g/h Methanol und 12 g/h Wasser eingespeist.
Von dem am Kopf der Kolonne erhaltenen Gasstrom wurden Proben genommen, die hinsichtlich ihrer Zusammensetzung analysiert wurden.

| Ergebnis: | Kohlendioxid | 71 % |
|-----------|--------------|------|
|           | Methylnitrit | 24 % |
|           | Methanol     | 5 %  |

Das am unteren Auslauf der Kolonne anfallende flüssige Produkt wurde gesammelt und hinsichtlich seiner Zusammensetzung analysiert.

| Ergebnis: | Wasser        | 42 % |
|-----------|---------------|------|
|           | Salpetersäure: | 58 % |

Methanol konnte nicht nachgewiesen werden.

Beispiel 5

Ein Kohlendioxid-Gasstrom von 10 l/h wurde mit einem Stickstoffdioxidgasstrom von 2,5 l/h (0,111 mol/h) gemischt, auf 25°C temperiert und am unteren Ende eines mit Raschig-Ringen befüllten und auf 25°C temperierten Rohres eingeleitet (Abmessungen: 1,6 cm Durchmesser, 50 cm Höhe).
Im Gegenstrom dazu wurden am oberen Ende des Reaktors 22 g/h Methanol eingespeist.
Von dem am Kopf der Kolonne erhaltenen Gasstrom wurden Proben genommen, die hinsichtlich ihrer Zusammensetzung analysiert wurden.

| Ergebnis: | Kohlendioxid | 84 % |
|-----------|--------------|------|
|           | Methylnitrit | 11 % |
|           | Methanol     | 5 %  |

Beispiel 6

Ein Kohlendioxid-Gasstrom von 10 l/h wurde mit einem Stickstoffdioxidgasstrom von 2,5 l/h (0,111 mol/h) gemischt, auf 30°C temperiert und am unteren Ende eines mit Raschig-Ringen befüllten und auf 30°C temperierten Rohres eingeleitet (Abmessungen: 1,6 cm Durchmesser, 50 cm Höhe).
Im Gegenstrom dazu wurden am oberen Ende des Reaktors 4,2 g/h Ethanol und 3 g/h Wasser eingespeist.
Von dem am Kopf der Kolonne erhaltenen Gasstrom wurden Proben genommen, die hinsichtlich ihrer Zusammensetzung analysiert wurden.

| Ergebnis: | Kohlendioxid | 82 % |
|-----------|--------------|------|
|           | Ethylnitrit  | 13 % |
|           | Ethanol      | 5 %  |

Das am unteren Auslauf der Kolonne anfallende flüssige Produkt wurde gesammelt und analysiert.

| Ergebnis: | Wasser | 45 % |
|---|---|---|
| | Salpetersäure | 55 % |

Ethanol konnte nicht nachgewiesen werden.

Beispiel 7

Der in Beispiel 6 beschriebene Versuch wurde wiederholt, wobei der Gasstrom und das Reaktionsrohr statt auf 30°C auf 40°C temperiert wurde.

| Ergebnis: | Kohlendioxid | 82 % |
|---|---|---|
| | Ethylnitrit | 12 % |
| | Ethanol | 6 % |

Das am unteren Auslauf der Kolonne anfallende flüssige Produkt wurde gesammelt und hinsichtlich seiner Zusammensetzung analysiert.

| Ergebnis: | Wasser | 45 % |
|---|---|---|
| | Salpetersäure | 55 % |

Ethanol konnte nicht nachgewiesen werden.

Vergleichsbeispiel 1

Ein Kohlendioxid-Gasstrom von 10 l/h wurde mit einem Stickstoffdioxidgasstrom von 2,5 l/h (0,111 Mol/h) gemischt und bei 25°C unter Rühren in 200 ml Methanol eingeleitet.
Bereits nach 3 Stunden konnte in dem entstehenden Methanol/Salpetersäure-Gemisch Methylnitrat nachgewiesen werden.

**Patentansprüche**

1. Verfahren zur Herstellung von $C_1$-$C_6$-Alkylnitriten durch Umsetzung von Stickstoffdioxid mit $C_1$-$C_6$-Alkoholen, dadurch gekennzeichnet, daß man in einer im Gegenstrom betriebenen Kolonne den Alkohol oder ein Alkohol/Wasser-Gemisch in den oberen Teil der Kolonne einspeist und das Stickstoffdioxid oder ein Stickstoffdioxid/Inertgas-Gemisch in den unteren Teil der Kolonne einspeist und das entstehende Alkylnitrit als Kopfprodukt und die mitentstehende Salpetersäure als Fußprodukt der Kolonne entnimmt.

2. Verfahren nach Anspruch 1, dadurch gekennzeichnet, daß geradkettige $C_1$-$C_4$-Alkohole eingesetzt werden.

3. Verfahren nach Anspruch 2, dadurch gekennzeichnet, daß Methanol oder Ethanol eingesetzt wird.

4. Verfahren nach Anspruch 1, dadurch gekennzeichnet, daß das molare Verhältnis von Alkohol zu Stickstoffdioxid 0,3 bis 1:1, bevorzugt 0,4 bis 0,8:1, besonders bevorzugt 0,5 bis 0,7:1 beträgt.

5. Verfahren nach Anspruch 1, dadurch gekennzeichnet, daß das molare Verhältnis von Wasser zu Stickstoffdioxid 0 bis 2:1, bevorzugt 0,2 bis 1,6:1, besonders bevorzugt 0,8 bis 1:1 beträgt.

6. Verfahren nach Anspruch 1, dadurch gekennzeichnet, daß man unter einem Druck von 0,5 bis 10 bar, bevorzugt 0,8 bis 8 bar, besonders bevorzugt 1 bis 5 bar arbeitet.

**7.** Verfahren nach Anspruch 1, dadurch gekennzeichnet, daß man bei einer Temperatur von 0 bis 90°C, bevorzugt 10 bis 80°C arbeitet.

**8.** Verfahren nach Anspruch 1, dadurch gekennzeichnet, daß das Volumenverhältnis von Inertgas zu Stickstoffdioxid, ausgedrückt in Nl/Nl 0 bis 5:1, bevorzugt 0,1 bis 3:1, besonders bevorzugt 0,2 bis 2:1 beträgt.

**9.** Verfahren nach Anspruch 1, dadurch gekennzeichnet, daß die Belastung der Reaktionskolonne mit den eingespeisten Gasen 10 bis 3000, bevorzugt 20 bis 2000, besonders bevorzugt 40 bis 1000 Nl/l freies Kolonnenvolumen pro Stunde beträgt.

**10.** Verfahren nach Anspruch 1, dadurch gekennzeichnet, daß der Alkohol oberhalb der Dosierung des Wassers in die Kolonne eingespeist wird.

## Claims

**1.** Process for the preparation of $C_1$-$C_6$-alkyl nitrites by reacting nitrogen dioxide with $C_1$-$C_6$ alcohols, characterized in that, in a countercurrent-operated column, the alcohol or an alcohol/water mixture is fed into the upper part of the column and the nitrogen dioxide or a nitrogen dioxide/inert gas mixture is fed into the lower part of the column and the resulting alkyl nitrite is removed from the column as top product and the nitric acid formed is removed from the column as bottom product.

**2.** Process according to Claim 1, characterized in that straight-chain $C_1$-$C_4$ alcohols are used.

**3.** Process according to Claim 2, characterized in that methanol or ethanol is used.

**4.** Process according to Claim 1, characterized in that the molar ratio of alcohol to nitrogen dioxide is 0.3 to 1:1, preferably 0.4 to 0.8:1, especially Preferably 0.5 to 0.7:1.

**5.** Process according to Claim 1, characterized in that the molar ratio of water to nitrogen dioxide is 0 to 2:1, preferably 0.2 to 1.6:1, especially preferably 0.8 to 1:1.

**6.** Process according to Claim 1, characterized in that the operation is carried out under a pressure of 0.5 to 10 bar, preferably 0.8 to 8 bar, especially preferably 1 to 5 bar.

**7.** Process according to Claim 1, characterized in that the operation is carried out at a temperature of 0 to 90°C, preferably 10 to 80°C.

**8.** Process according to Claim 1, characterized in that the volume ratio of inert gas to nitrogen dioxide, expressed in l (STP)/l (STP), is 0 to 5:1, preferably 0.1 to 3:1, especially preferably 0.2 to 2:1.

**9.** Process according to Claim 1, characterized in that the loading of the reaction column with the fed-in gases is 10 to 3000, preferably 20 to 2000, especially preferably 40 to 1000 l (STP)/l of free column volume per hour.

**10.** Process according to Claim 1, characterized in that the alcohol is fed into the column above the site of metered addition of the water.

## Revendications

**1.** Procédé de préparation de nitrites d'alkyle $C_1$-$C_6$ par réaction de dioxyde d'azote avec des alcools $C_1$-$C_6$, caractérisé en ce qu'on introduit dans une colonne fonctionnant à contre-courant l'alcool ou un mélange alcool/eau à la partie supérieure de la colonne et qu'on introduit le dioxyde d'azote ou un mélange dioxyde d'azote/gaz inerte dans la partie inférieure de la colonne et on prélève le nitrite d'alkyle formé comme produit de tête et l'acide nitrique coproduit comme produit de pied de la colonne.

**2.** Procédé selon la revendication 1, caractérisé en ce qu'on utilise des alcools à chaîne droite $C_1$-$C_4$.

**3.** Procédé selon la revendication 2, caractérisé en ce qu'on utilise du méthanol ou de l'éthanol.

4.  Procédé selon la revendication 1, caractérisé en ce que le rapport molaire de l'alcool au dioxyde d'azote est 0,3 à 1:1, de préférence de 0,4 à 0,8:1, surtout 0,5 à 0,7:1.

5.  Procédé selon la revendication 1, caractérisé en ce que le rapport molaire de l'eau au dioxyde d'azote est 0 à 2:1, de préférence 0,2 à 1,6:1, surtout 0,8 à 1:1.

6.  Procédé selon la revendication 1, caractérisé en ce qu'on travaille sous une pression de 0,5 à 10 bar, de préférence 0,8 à 8 bar, surtout 1 à 5 bar.

7.  Procédé selon la revendication 1, caractérisé en ce qu'on travaille à une température de 0 à 90°C, de préférence 10 à 80°C.

8.  Procédé selon la revendication 1, caractérisé en ce que le rapport volumique du gaz inerte au dioxyde d'azote, exprimé en NI/NI est de 0 à 5:1, de préférence 0,1 à 3:1, surtout 0,2 à 2:1.

9.  Procédé selon la revendication 1, caractérisé en ce que la charge de la colonne de réaction avec les gaz alimentés est de 10 à 3000, de préférence 20 à 2000, surtout à 40 à 1000 NI/I de volume de colonne libre par heure.

10. Procédé selon la revendication 1, caractérisé en ce qu'on introduit l'alcool après le dosage de l'eau dans la colonne.